# EUROPEAN PATENT SPECIFICATION

(11) **EP 0 444 728 B1**
(45) Date of publication and mention of the grant of the patent: **10.09.1997**
(21) Application number: 91200164.1
(22) Date of filing: 28.01.1991
(51) Int. Cl.: A61M 25/00, B29C 70/82

(54) **Method of manufacturing a catheter with braided sheath**
Verfahren zur Herstellung eines Katheters mit geflochtener Hülse
Procédé de fabrication d'un cathéter avec gaine tressée

(30) Priority: 26.02.1990 NL 9000458
(43) Date of publication of application: 04.09.1991
(73) Proprietor: CORDIS EUROPA N.V., NL-9301 LJ Roden (NL)
(72) Inventor: Wijkamp, Arnoldus Cornelius Maria, NL-9302 AW Roden (NL); Antoni, Robert, NL-9725 CD Groningen (NL); Venema, Hendrik Jan, NL-9301 PD Roden (NL); Griep, Wilhelmus Antonius Maria, NL-9301 PK Roden (NL); Mulder, Rudolf, NL-9717 AC Groningen (NL)
(74) Representative: 't Jong, Bastiaan Jacobus

(56) References cited:
- GB-A- 1 086 822
- US-A- 3 965 909
- US-A- 4 577 543

## Description

The invention relates to a method for manufacturing a braided catheter as defined in the introductory part of claim 1.

Such a method is known from US-A-4 577 543. With this known method after arranging the braiding, the basic body is passed through a heated sizing die, causing the basic body to become tacky so that the braided threads are held in place thereon.

An object of the present invention is to provide a method of the kind set forth above, for manufacturing a catheter with a smaller outer diameter, especially by reducing the wall thickness.

With the method according to the invention as characterized in claim 1 this object is achieved. The diameter of the die can be chosen such that the braiding threads come to lie at a smaller effective diameter. The resulting slack in the thread is directly taken up as a result of the tension in the braiding, so that the threads of the reinforcing sheats remain taut after embedding. Accordingly it is possible to significantly reduce the diameter of the braiding and accordingly the diameter of the outer layer and accordingly the diameter of the finished catheter.

The invention is further elucidated in the following description with reference to the annexed figures.

Fig. 1 shows a catheter.

Fig. 2 shows a partially simplified perspective view of a device according to the invention.

Fig. 3 shows a partially perspective view according to III in fig. 2, with components drawn at a distance from each other in vertical direction.

Fig. 4 shows a partially broken away perspective detail view according to IV in fig. 3.

Fig. 5 shows a partial section of a catheter semi-manufacture manufactured using the invention.

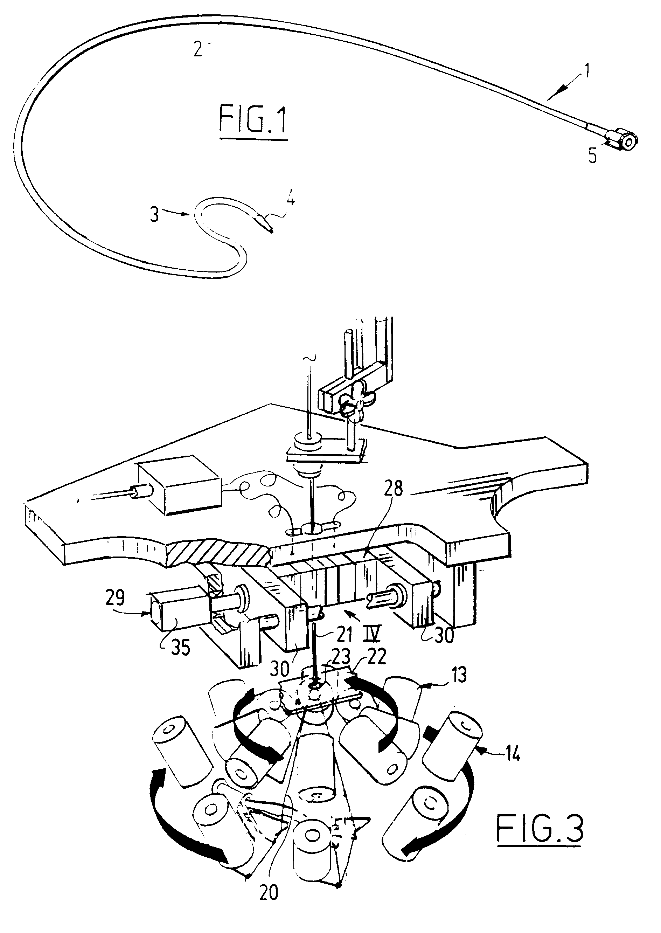

A catheter 1 as shown in fig. 1 is manufactured in a number of steps in the known manner. First a hose-like basic body of flexible material is manufactured, usually by extruding it around a metal carrying thread. A reinforcing sheath of thin steel wire is subsequently braided around this hose-like basic body. An outer layer of flexible material is then arranged around this reinforcing sheath in the usual manner by extruding it therearound. This base material is manufactured in great lengths.

For finishing to a catheter 1, lengths are cut off the manufactured base material which each form a basic portion 2 for a catheter 1. Arranged on an end of this basic portion 2 is a hose-like end portion 3 which does not normally have a braided reinforcing sheath and of which the extremity 4 is pointed. On the other end of the basic portion 2 a connecting element 5 is arranged. The metal carrying thread is of course removed and leaves behind in the basic portion 2 a central channel through which a liquid can be transported. The above described manufacturing method for a catheter is per se known.

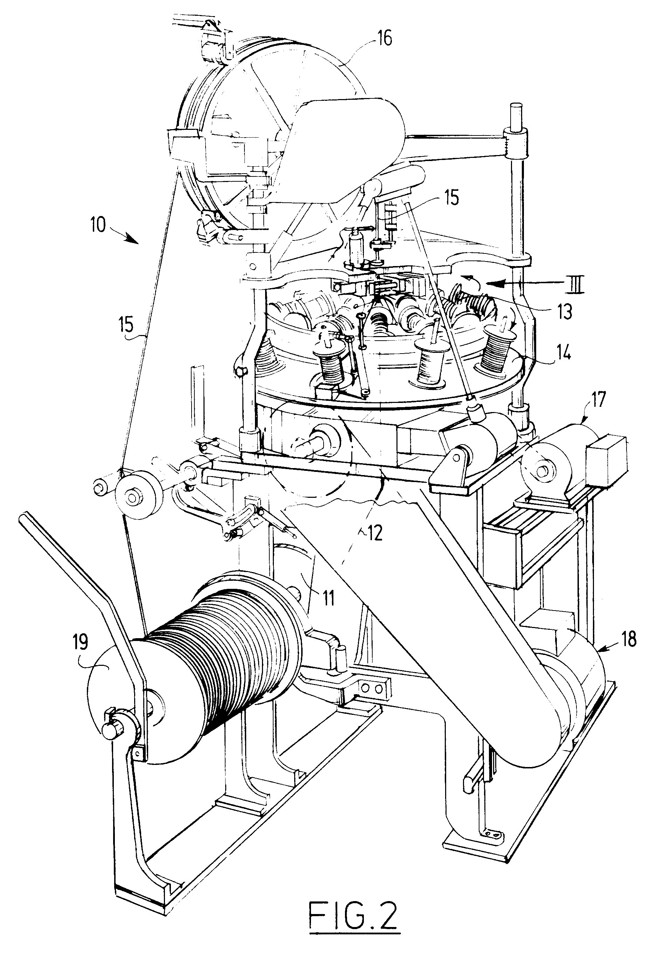

Fig. 2 shows a device of the generally known type which is altered to a device according to the invention, that is used for braiding the reinforcing sheath. Placed in this device 10 is a supply reel 11 onto which is wound a great length of the hose-like basic body. As stated, this basic body can be extruded around a metal thread in a previous method step.

The hose-like basic body is taken from the supply reel 11 and guided via the path indicated with dashed lines in fig. 2 upward through the device 10. The device 10 comprises at the top a transporting wheel 16 around which the basic body is guided a number of times. From the transporting wheel 16 the basic body is guided to a winding reel 19. During operation of the device 10 the transporting wheel 16 and the associated transporting means are driven by a drive device 17 such that the basic body is wound off the supply reel 11, guided through the machine and wound up on the winding reel 19.

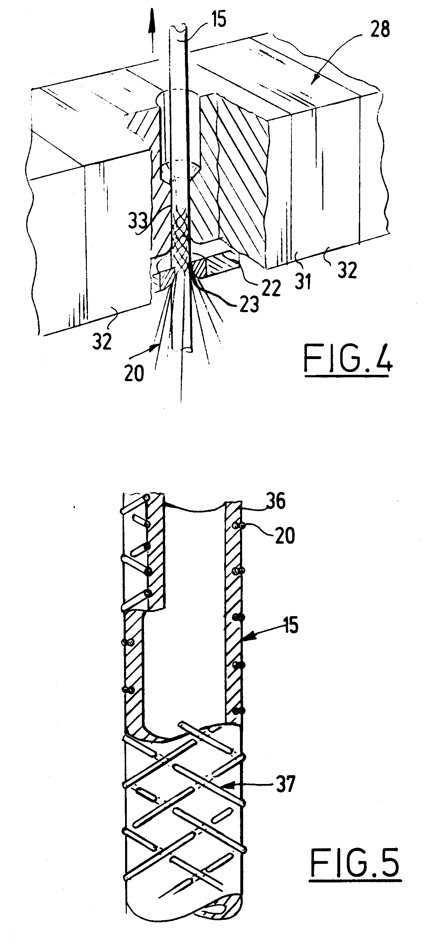

A portion of the path 12 along which the basic body is guided coincides with a line of axis about which two thread spool carriers 13 and 14 are mounted for opposing rotation. The thread spool carriers 13 and 14 are driven using a driving 18. The thread spool carriers 13 and 14 bear a number of spools with a thin metal thread which, during the rotation of the carriers 13 and 14, are wound around the basic body transported in its lengthwise direction. The position where the threads 20 from the spools come together and are wound around the basic body, will hereafter be referred to as the braiding position. In this braiding position is situated a braiding guide 22 with a hole 23 that runs out downward. In the zone past the braiding guide 22, designated in fig. 3 by 21, the basic body is thus provided with a reinforcing sheath wound or braided around it and lying on the outer surface.

The device according to the invention comprises embedding means 28 arranged directly following the braiding position. For the sake of clarity in the figure the embedding means 28 are shown in fig. 3 at a large interval above the braiding position defined by the braiding guide 22. In reality the embedding means 28 are situated as closely as possible above the braiding position. This is shown in fig. 4.

The embedding means 28 comprise a drawing block 31 with a guide channel 33 disposed coaxially to the previously mentioned line of axis and having a calibrated diameter. This diameter is smaller than the outer diameter of the reinforcing sheath in the zone 21. By passing through the calibrated channel 33 the reinforcing sheath is therefore pressed or embedded into the basic body.

Fig. 5 shows the assembly 15 after it has passed through the drawing block 31. The threads 20 of the reinforcing sheath 37 are, as can be seen, at least partially embedded in the basic body 36. The outer diameter of the assembly 15 is therefore reduced relative to the assembly in the zone 21. The at least one outer layer yet to be applied to the assembly 15, can therefore, with a more or less fixed minimum thickness determined by the manufacturing conditions, have a smaller outer diameter than if the sheath 37 were not to be embedded.

The drawing block 31 is clamped into the machine using a clamping mechanism 29. This comprises two jaws 30 slidable relative to the frame of the machine which can be constrained towards one another using a pneumatic piston/cylinder unit 35. Clamped in with the drawing block 31 on either side thereof are heating units 32. These heating units 32 heat the drawing block 31 so that the threads 20 of the reinforcing sheath are heated in the channel 33 and are melted into the basic body 36.

As noted earlier the embedding means 28 are arranged as closely as possible above the braiding position or above the braiding guide 22. The thread tension maintained in the threads 20 during braiding can hereby be further transmitted into the channel 33, where the threads are constrained into a smaller diameter and where therefore space in the threads could be created. As a result of the thread tension this space is taken up, so that the threads 20 remain taut in the embedded braided sheath.

To allow the thread tension to continue acting as well as possible into the embedding zone, the braiding guide 22 is moreover embodied with a minimum axial length. Although this is not shown in detail, the braiding guide 22 is self-centering relative to the drawing block 31, in order that the opening 23 be adjusted precisely coaxially to the channel 33. Hereby ensured is that the sheath is embedded into the basic body uniformly over the whole periphery.

By embedding the threads 20 of the sheath 37 the further advantage is also achieved that during the subsequent extruding of the at least one outer layer therearound, the reinforcing sheath is well fixed relative to the basic body and therefore cannot roll up.

## Claims

1. Method for manufacturing a catheter comprising manufacturing a hose-like basic body of flexible material, braiding a reinforcing sheath around said basic body, arranging at least one outer layer of flexible material around said reinforcing sheath and finishing of the obtained assembly to a catheter (1), whereby said reinforcing sheath is at least partially embedded in said basic body by passing through a die, the reinforcing sheath is braided from metal threads (20) and is embedded in the basic body as it is advanced gradually during braiding, **characterized in that** the embedding takes place at a position (33) directly following the position (23) where the braiding takes place, such that slack in the threads due to the embedding is taken up as a result of thread tension during braiding.

## Patentansprüche

1. Verfahren zur Herstellung eines Katheters mit den folgenden Schritten: es wird ein schlauchartiger Grundkörper aus flexiblem Material hergestellt; es wird eine Verstärkungshülse um den Grundkörper herumgeflochten; es wird wenigstens eine äußere Schicht aus flexiblem Material um die Verstärkungshülse herum angeordnet, und der so erhaltene Aufbau wird zu einem Katheter (1) vervollständigt, wobei die Verstärkungshülle wenigstens teilweise in dem Grundkörper eingebettet wird, indem sie durch ein Formgesenk hindurchgeführt wird, und die Verstärkungshülse wird aus Metallfäden (20) geflochten und im Grundkörper eingebettet, wenn dieser graduell während des Flechtvorgangs vorgeschoben wird,
dadurch gekennzeichnet, daß die Einbettung an einer Stelle (33) direkt hinter der Stelle (23) stattfindet, wo der Flechtvorgang stattfindet, so daß ein Durchhang in den Fäden infolge der Einbettung aufgrund der Fadenspannung während der Umflechtung aufgenommen wird.

## Revendications

1. Procédé de fabrication d'un cathéter, comprenant la fabrication d'un corps de base du type tuyau en matériau souple, le tressage d'une gaine de renforcement autour dudit corps de base, l'agencement d'au moins une couche extérieure de matériau souple autour de ladite gaine de renforcement et la finition de l'ensemble obtenu en un cathéter (1), ladite gaine de renforcement étant au moins partiellement insérée dans ledit corps de base au moyen d'un passage dans une filière, la gaine de renforcement étant tressée à partir de fils métalliques (20) et étant insérée dans le corps de base en étant avancée progressivement pendant le tressage, caractérisée en ce que l'insertion est effectuée à un emplacement (33) suivant directement l'emplacement (23) où le tressage est effectué, de sorte que le relâchement des fils qui est dû à l'insertion est repris du fait de la tension de fil pendant le tressage.
